# EUROPEAN PATENT APPLICATION

(11) **EP 4 821 583 A1**
(43) Date of publication of application: **14.10.2026**
(21) Application number: 26164910.7
(22) Date of filing: 16.03.2026
(51) Int. Cl.: B01D 53/047, B01D 53/04

(54) **DAC APPARATUS**

(30) Priority: 19.03.2025 JP 2025045894
(71) Applicant: Honda Motor Co., Ltd., Toyko 105-8404 (JP)
(72) Inventor: INABA, Toshiyuki, Saitama, 351-0193 (JP)
(74) Representative: Weickmann & Weickmann PartmbB

(57) **Abstract**

DAC apparatus (100) configured to recover CO₂ in atmosphere, includes: adsorption-chamber (1) provided with adsorbent for adsorbing or absorbing CO₂; atmospheric-control-valve (1a,1b) configured to open adsorption-chamber to atmosphere or to seal adsorption-chamber from atmosphere; vacuum-piping (4) connecting adsorption-chamber with vacuum-pump (5); vacuum-control-valve (4a) provided on vacuum-piping; heating-device (7) configured to heat adsorbent; and controller (10) configured to control atmospheric-control-valve, vacuum-control-valve, and heating-device to alternately perform adsorption-step and desorption-step, adsorption-step opening adsorption-chamber to atmosphere, prohibiting flow of gas to vacuum-piping, and adsorbing CO₂ in atmosphere to adsorbent, desorption-step sealing adsorption-chamber, permitting flow of gas to vacuum-piping, and heating adsorbent to desorb CO₂ from adsorbent. Supply-piping (9) to which CO₂ is supplied is further connected with adsorption-chamber. Supply-control-valve (9a) is provided on Supply-piping. Controller further controls Supply-control-valve to supply adsorption-chamber with CO₂ in desorption-step.

## Description

### BACKGROUND OF THE INVENTION

### Field of the Invention

The present invention relates to a direct air capture (DAC) apparatus that recovers carbon dioxide (CO₂) in the atmosphere.

### Description of the Related Art

For the purpose of mitigating climate changes, research and development have been conducted on DAC technology for recovering CO₂, which is a main greenhouse gas. As such a DAC technology, an apparatus that recovers CO₂ in the atmosphere using an adsorbent for adsorbing or absorbing CO₂ is known. For example, in the apparatus of JP 2024-048937 A, after the atmosphere is caused to circulate into an adsorption chamber in which an adsorbent is provided and to adsorb CO₂ in the atmosphere to the adsorbent, the adsorbent is heated to desorb CO₂ from the adsorbent. When the pressure inside the adsorption chamber reaches a threshold value, a desorption gas having a high CO₂ concentration is recovered from the inside of the adsorption chamber using a vacuum pump.

However, as in the apparatus described in JP 2024-048937 A, by merely heating the adsorbent at the time of desorption, the temperature rise of the adsorbent is delayed. Hence, it takes a long time for desorption, and CO₂ recovery efficiency as the entire apparatus may be lowered.

### SUMMARY OF THE INVENTION

An aspect of the present invention is a DAC apparatus configured to recover CO₂ in the atmosphere, including: an adsorption chamber provided with an adsorbent for adsorbing or absorbing CO₂; an atmospheric control valve configured to open the adsorption chamber to the atmosphere or to seal the adsorption chamber from the atmosphere; a vacuum piping connecting the adsorption chamber with a vacuum pump; a vacuum control valve provided on the vacuum piping; a heating device configured to heat the adsorbent; and a controller configured to control the atmospheric control valve, the vacuum control valve, and the heating device to alternately perform an adsorption step and a desorption step, the adsorption step opening the adsorption chamber to the atmosphere, prohibiting a flow of gas to the vacuum piping, and adsorbing CO₂ in the atmosphere to the adsorbent, the desorption step sealing the adsorption chamber, permitting the flow of the gas to the vacuum piping, and heating the adsorbent to desorb CO₂ from the adsorbent. A supply piping to which CO₂ is supplied is further connected with the adsorption chamber. A supply control valve is provided on the supply piping. The controller further controls the supply control valve to supply the adsorption chamber with CO₂ in the desorption step.

Another aspect of the present invention is a DAC apparatus configured to recover CO₂ in the atmosphere, including: an adsorption chamber provided with an adsorbent for adsorbing or absorbing CO₂; an atmospheric control valve configured to open the adsorption chamber to the atmosphere or to seal the adsorption chamber from the atmosphere; a vacuum piping connecting the adsorption chamber with a vacuum pump; a vacuum control valve provided on the vacuum piping; a heating device configured to heat the adsorbent; and a controller configured to control the atmospheric control valve, the vacuum control valve, and the heating device to alternately perform an adsorption step and a desorption step, the adsorption step opening the adsorption chamber to the atmosphere, prohibiting a flow of gas to the vacuum piping, and adsorbing CO₂ in the atmosphere to the adsorbent, the desorption step sealing the adsorption chamber, permitting the flow of the gas to the vacuum piping, and heating the adsorbent to desorb CO₂ from the adsorbent. The controller: reduces a pressure of the adsorption chamber to a predetermined pressure by sealing the adsorption chamber and permitting the flow of the gas to the vacuum piping when the adsorption step ends; and controls the heating device to start heating the adsorbent before the pressure of the adsorption chamber reaches the predetermined pressure in such a manner that the temperature of the adsorbent starts to rise at a timing when the pressure of the adsorption chamber reaches the predetermined pressure.

### BRIEF DESCRIPTION OF THE DRAWINGS

The objects, features, and advantages of the present invention will become clearer from the following description of embodiments in relation to the attached drawings, in which:
FIG. 1 is a block diagram schematically illustrating an example of a piping configuration of a DAC apparatus according to an embodiment of the present invention;
FIG. 2 is a block diagram schematically illustrating an example of a control configuration of the DAC apparatus;
FIG. 3 is a time chart for describing temperature changes of an adsorbent during a desorption step;
FIG. 4A is a time chart for describing temperature changes of the adsorbent when CO₂ is supplied to a reactor in the desorption step;
FIG. 4B is a time chart for describing temperature changes of the adsorbent when heating of the adsorbent is started in a deaeration step;
FIG. 5A is a flowchart illustrating an example of processing in the deaeration step performed by a controller in FIG. 2; and
FIG. 5B is a flowchart illustrating another example of processing in the deaeration step performed by the controller in FIG. 2.

### DETAILED DESCRIPTION OF THE INVENTION

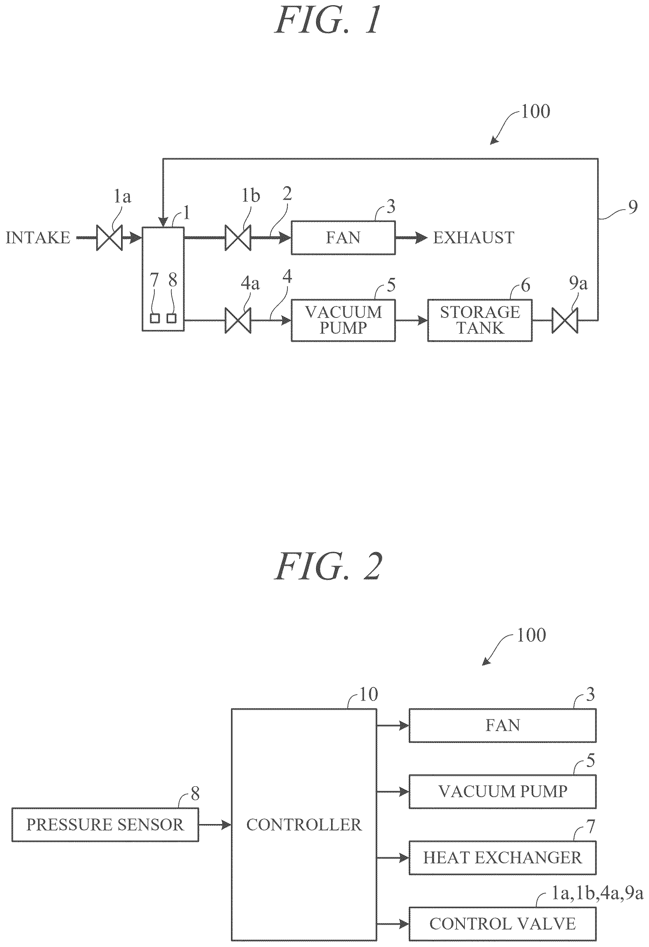

Hereinafter, embodiments of the present invention will be described with reference to FIGS. 1 to 5B. FIG. 1 is a block diagram schematically illustrating an example of a piping configuration of a DAC apparatus 100 according to an embodiment of the present invention. As illustrated in FIG. 1, the DAC apparatus 100 mainly includes a reactor 1, in which an adsorbent for adsorbing or absorbing CO₂ is provided.

The reactor 1 includes a housing in which a sealed space (an adsorption chamber) can be formed, and the adsorbent is provided in the adsorption chamber of the reactor 1. As the adsorbent, a solid material constituted to adsorb CO₂ at normal temperature and normal pressure and to desorb CO₂ as the temperature rises can be used. For example, an amine-based solid absorbent in which an amine-based compound is carried on an appropriate carrier can be used. The adsorbent is constituted in an appropriate granular shape, is filled in a filter cartridge or the like having a mesh structure, for example, and is accommodated in the adsorption chamber of the reactor 1.

Atmospheric control valves 1a and 1b, each of which opens the reactor 1 to the atmosphere or seals the reactor 1 from the atmosphere, are provided for the reactor 1. The atmospheric control valves 1a and 1b may be provided on piping that connects the adsorption chamber of the reactor 1 with an external space, or may be provided as a part of a housing (a wall surface) of the reactor 1. When the atmospheric control valves 1a and 1b are open, the adsorption chamber of the reactor 1 is open to the atmosphere. When the atmospheric control valves 1a and 1b are closed, the adsorption chamber of the reactor 1 is sealed. The atmospheric control valve 1a and the atmospheric control valve 1b are provided to face each other with the adsorption chamber interposed between them.

A fan 3 for circulating the atmosphere into the adsorption chamber of the reactor 1 is connected with the reactor 1 through appropriate piping (atmospheric piping) 2 and one of the atmospheric control valves 1a and 1b (the atmospheric control valve 1b in the illustrated example). In the illustrated example, when the atmospheric control valves 1a and 1b are open and the fan 3 is driven, the atmosphere is taken into the reactor 1 through the atmospheric control valve 1a, and the atmosphere is exhausted from the reactor 1 through the atmospheric control valve 1b, the atmospheric piping 2, and the fan 3. Thus, when the atmosphere circulates into the reactor 1, CO₂ in the atmosphere is adsorbed to the adsorbent in the adsorption chamber (an adsorption step). The atmospheric control valves 1a and 1b and the atmospheric piping 2 are each configured to have a relatively large diameter so as to enable a large amount of atmosphere to circulate into the reactor 1. A plurality of the atmospheric control valves 1a and a plurality of the atmospheric control valves 1b may be provided.

Vacuum piping 4 is further connected with the reactor 1. The vacuum piping 4 connects the adsorption chamber of the reactor 1 with an intake port of a vacuum pump 5 for CO₂ recovery. On the vacuum piping 4, a vacuum control valve 4a, which permits or prohibits a flow of gas in the vacuum piping 4, that is, the flow of gas from the reactor 1 to the vacuum piping 4, is provided. The vacuum control valve 4a may be made up of an on-off valve such as a ball valve, an adjustment valve such as a butterfly valve, or a combination of them. A storage tank 6 is connected with an exhaust port of the vacuum pump 5 through appropriate piping.

In the reactor 1, a heating device that heats the adsorbent and a cooling device that cools the adsorbent are also provided. For example, a heat exchanger 7, which heats or cools the adsorbent using a heat medium, is provided. In the reactor 1, a pressure sensor 8, which detects pressure P of the reactor 1 (the adsorption chamber), is also provided.

When the adsorption step ends, the atmospheric control valves 1a and 1b are closed, and in addition, the vacuum control valve 4a is opened. When the vacuum pump 5 is driven, the gas in the adsorption chamber of the reactor 1 is sucked out by the vacuum pump 5 through the vacuum piping 4. Thus, the inside of the adsorption chamber of the reactor 1 is deaerated, and the pressure P of the reactor 1 becomes lower than that at the time of adsorption (a deaeration step).

When the deaeration step ends, the adsorbent starts to be heated by the heat exchanger 7 in a state in which the pressure P of the reactor 1 is lower than that at the time of adsorption. Thus, a temperature T of the adsorbent rises to be higher than that at the time of adsorption in a state in which the pressure P of the reactor 1 is lower than that at the time of adsorption. The CO₂ that has been adsorbed to the adsorbent is desorbed, and the desorption gas mainly containing the desorbed CO₂ is sucked from the reactor 1, is stored in the storage tank 6, and is recovered (a desorption step).

In heating the adsorbent in the desorption step, if the temperature of the adsorbent rises in a state in which oxygen is present in the adsorption chamber, the adsorbent may be subject to oxidation deterioration. By performing the deaeration step before the desorption step accompanied by the temperature rise of the adsorbent starts, and removing the residual atmosphere in the adsorption chamber containing oxygen, it becomes possible to prevent the oxidation deterioration of the adsorbent.

The deaeration step may be performed using the vacuum pump 5 for CO₂ recovery. However, a vacuum pump (a vacuum pump for deaeration) different from the vacuum pump 5 may be connected with the adsorption chamber of the reactor 1 through vacuum piping (vacuum piping for deaeration) different from the vacuum piping 4, and then the deaeration step may be performed using such a vacuum pump for deaeration. In this case, it is possible to exhaust the residual atmosphere that has been deaerated from the inside of the adsorption chamber of the reactor 1 through such a vacuum pump for deaeration without guiding the residual atmosphere to the storage tank 6, so that the concentration of CO₂ recovered into the storage tank 6 can be further increased.

After the desorption step ends and before the adsorption step starts, a cooling step of cooling the adsorbent in the adsorption chamber of the reactor 1 is performed. When the atmosphere containing oxygen circulates into the adsorption chamber in a state in which the adsorbent has a high temperature, the adsorbent may be subject to the oxidation deterioration. By performing the cooling step of cooling the adsorbent before the adsorption step of causing the atmosphere to circulate into the adsorption chamber, it becomes possible to prevent the oxidation deterioration of the adsorbent. In the cooling step, processing may wait until the temperature T of the adsorbent decreases to fall within a temperature range in which an oxidation reaction does not proceed, but the adsorbent in the adsorption chamber may be cooled by the heat exchanger 7. In this case, the time necessary for the cooling step can be shortened, so that the DAC apparatus 100 can be efficiently operated.

However, if the temperature rise of the adsorbent is delayed at the start timing of the desorption step, a long time is needed for the desorption step, and the operation efficiency is lowered. Hence, the CO₂ recovery efficiency of the entire apparatus may be lowered. Therefore, in the present embodiment, the DAC apparatus is configured as follows in such a manner that the temperature rise of the adsorbent at the start timing of the desorption step is accelerated, so that the CO₂ recovery efficiency of the entire apparatus can be improved.

### Temperature Rise of Adsorbent due to Adsorption Heat

As illustrated in FIG. 1, the DAC apparatus 100 further includes supply piping 9, which connects the storage tank 6 with the reactor 1. On the supply piping 9, a supply control valve 9a, which permits or prohibits the flow of gas in the supply piping 9, that is, the flow of gas from the storage tank 6 to the reactor 1, is provided. The internal pressure of the storage tank 6 is higher than the atmospheric pressure. Therefore, when the supply control valve 9a is caused to open in the deaeration step in which the reactor 1 (the adsorption chamber) is depressurized by the vacuum pump 5 or in the desorption step, some amount of CO₂ stored in the storage tank 6 is supplied to the reactor 1 through the supply piping 9.

FIG. 2 is a block diagram schematically illustrating an example of a control configuration of the DAC apparatus 100. As illustrated in FIG. 2, the DAC apparatus 100 includes a controller 10, which controls each unit of the DAC apparatus 100 illustrated in FIG. 1. The controller 10 is made up of a computer including a CPU, a ROM, a RAM, an I/O interface, and other peripheral circuits. The pressure sensor 8 is connected with the controller 10, and a signal indicating a detection value of the pressure sensor 8 is input into the controller 10. In addition, the fan 3, the vacuum pump 5, the heat exchanger 7, and the control valves (the atmospheric control valves 1a and 1b, the vacuum control valve 4a, and the supply control valve 9a) are included in the DAC apparatus 100, and each unit of the DAC apparatus 100 is connected with the controller 10. A control signal is transmitted from the controller 10 to each unit of the DAC apparatus 100.

The controller 10 drives the fan 3 and the vacuum pump 5 to start the heat exchanger 7, and then controls each unit of the DAC apparatus 100 in such a manner that the adsorption step and the desorption step are alternately performed in the reactor 1. From the viewpoint of operation efficiency, the adsorption step continues for a predetermined time so that CO₂ is adsorbed to the adsorbent to a predetermined proportion (for example, approximately 70%) of the adsorption capacity. The deaeration step continues until pressure P of the reactor 1 decreases to be equal to or lower than predetermined pressure P1 corresponding to final ultimate pressure of the vacuum pump 5. The desorption step continues for a predetermined time so that after the temperature T of the adsorbent reaches a desorption temperature T0 (for example, approximately 100 degrees Celsius) of CO₂, the CO₂ that has been adsorbed to the adsorbent in the adsorption step is desorbed to have a predetermined proportion (for example, approximately 30%) of the adsorption capacity from the viewpoint of operation efficiency. The cooling step continues until the temperature T of the adsorbent decreases to be equal to or lower than a predetermined temperature at which the oxidation reaction does not proceed.

More specifically, in the adsorption step, the controller 10 opens the atmospheric control valves 1a and 1b to cause the atmosphere to circulate into the reactor 1, and also closes the vacuum control valve 4a to prohibit the flow of gas from the reactor 1 to the vacuum piping 4. In the deaeration step, the atmospheric control valves 1a and 1b are closed to seal the reactor 1, and in addition, the vacuum control valve 4a is open to permit the flow of gas from the reactor 1 to the vacuum piping 4. In the desorption step, in a state in which the atmospheric control valves 1a and 1b are closed to seal the reactor 1, and in addition, the vacuum control valve 4a is open to permit the flow of gas from the reactor 1 to the vacuum piping 4, the heat exchanger 7 is controlled to heat the adsorbent.

The deaeration step ends, and when the desorption step starts, the controller 10 temporarily opens the supply control valve 9a to supply some amount of CO₂ stored in the storage tank 6 to the reactor 1 through the supply piping 9. More specifically, in a state in which the atmospheric control valves 1a and 1b are closed to seal the reactor 1, and in addition, the vacuum control valve 4a is open to permit the flow of gas from the reactor 1 to the vacuum piping 4, the controller 10 temporarily opens the supply control valve 9a, and also controls the heat exchanger 7 to heat the adsorbent.

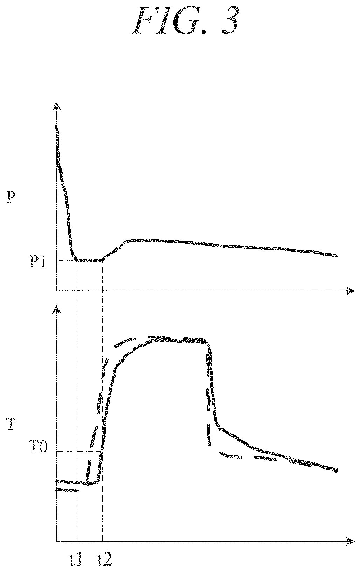

FIG. 3 is a time chart for describing temperature changes of the adsorbent during the desorption step, and illustrates the pressure P of the reactor 1, the temperature T (a solid line) of the adsorbent, and the temperature (a broken line) of the heat medium of the heat exchanger 7. The pressure P of the reactor 1 is normal pressure (atmospheric pressure) during the adsorption step. When the deaeration step starts, the pressure P decreases to the predetermined pressure P1 corresponding to the final ultimate pressure of the vacuum pump 5. That is, the pressure P of the reactor 1 during the deaeration step and the desorption step is a negative pressure lower than the atmospheric pressure, and the vacuum degree in the adsorption chamber increases as the pressure P in FIG. 3 decreases.

As illustrated in FIG. 3, when the pressure P of the reactor 1 reaches the predetermined pressure P1 at time t1, the adsorbent immediately starts to be heated, and the temperature (the broken line) of the heat medium of the heat exchanger 7 rises, but it takes a long time until the temperature T (the solid line) of the adsorbent starts to rise. In this case, it takes a long time until the temperature T of the adsorbent reaches the desorption temperature T0 of CO₂ at time t2.

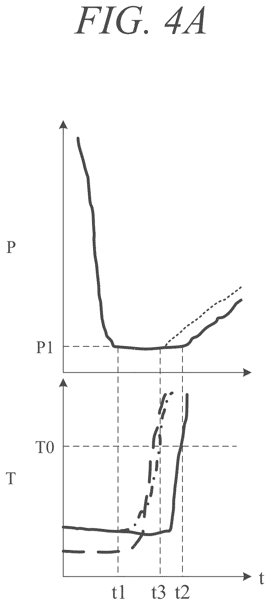

FIG. 4A is a time chart for describing temperature changes of the adsorbent when CO₂ is supplied to the reactor 1 in the desorption step, and illustrates the temperature T (a dashed line) of the adsorbent when CO₂ is supplied. As illustrated in FIG. 4A, when the pressure P of the reactor 1 reaches the predetermined pressure P1 at time t1, the adsorbent immediately starts to be heated, and in addition, some amount of CO₂ stored in the storage tank 6 is supplied to the reactor 1 through the supply piping 9. In this case, the temperature T (a dashed line) of the adsorbent starts to rise substantially at the same time as the temperature (a broken line) of the heat medium of the heat exchanger 7 rises, and the temperature T of the adsorbent reaches the desorption temperature T0 of CO₂ at time t3, which is earlier than time t2.

Adsorption equilibrium where the adsorption rate at which CO₂ is adsorbed to the adsorbent is equal to the desorption rate at which CO₂ is desorbed from the adsorbent is inclined to the adsorption side as the partial pressure of CO₂ in the adsorption chamber of the reactor 1 increases. By supplying the reactor 1 with CO₂ of an extremely high concentration stored in the storage tank 6, the partial pressure of CO₂ in the adsorption chamber increases. In this case, the adsorption equilibrium is inclined to the adsorption side, and thus CO₂ is adsorbed to the adsorbent. The temperature of the adsorbent itself rises due to the adsorption heat.

In this manner, by supplying CO₂ to the reactor 1 at the start timing of the desorption step and raising the temperature of the adsorbent itself due to the adsorption heat to accelerate the temperature rise, it becomes possible to shorten the time to reach the desorption temperature T0 of CO₂, that is, the time necessary for the desorption step, so that the CO₂ recovery efficiency of the entire apparatus can be improved. In addition, energy consumption necessary for heating the adsorbent with the heat exchanger 7 can be reduced. Further, by supplying CO₂ stored in the storage tank 6, a simple and waste-free apparatus configuration is achievable, and pulsation of the supply gas (CO₂) can be prevented.

### Earlier Heating Start Timing of Adsorbent

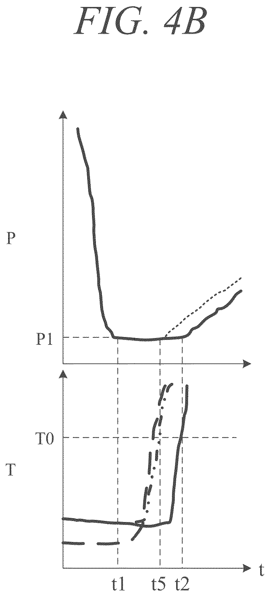

FIG. 4B is a time chart for describing temperature changes of the adsorbent when heating of the adsorbent is started in the deaeration step, and illustrates the temperature T (a dashed line) of the adsorbent and the temperature (a two-dot chain line) of the heat medium of the heat exchanger 7 when heating is started in the deaeration step. As illustrated in FIG. 4B, the adsorbent starts to be heated in the deaeration step at time t4, which is earlier than time t1 when the pressure P of the reactor 1 reaches the predetermined pressure P1. In this case, the pressure P of the reactor 1 reaches the predetermined pressure P1, and the deaeration step ends. After the desorption step starts, the temperature T of the adsorbent reaches the desorption temperature T0 of CO₂ at time t5, which is earlier than time t2.

For example, when the pressure P of the reactor 1 reaches predetermined heating start pressure P2, which is higher than the predetermined pressure P1, the adsorbent starts to be heated. Thus, it becomes possible to start heating the adsorbent at an appropriate timing in the deaeration step. The heating start pressure P2 is determined beforehand by experiment so that the temperature of the adsorbent starts to rise when the pressure P of the reactor 1 reaches the predetermined pressure P1. Such heating start pressure P2 may be set in accordance with the current temperature T of the adsorbent in such a manner that as the temperature T of the adsorbent decreases, the pressure increases, and as the temperature T of the adsorbent increases, the pressure decreases to be closer to the predetermined pressure P1.

In a case where the temperature T of the adsorbent is low and it is necessary to take time to raise the temperature and reach the desorption temperature T0 of CO₂, it is possible to start heating the adsorbent earlier by setting the heating start pressure P2 to be high. In a case where the heating start pressure P2 is set in accordance with the temperature T of the adsorbent, a temperature sensor for detecting the temperature T of the adsorbent is provided in the reactor 1, and the heating start pressure P2 is set in accordance with a detection value of the temperature sensor. A temperature sensor that detects the temperature of the reactor 1 (the adsorption chamber) having a correlation with the temperature T of the adsorbent may be used. The temperature T of the adsorbent in the deaeration step immediately after the adsorption step ends has a correlation with the outside air temperature, and thus a temperature sensor that detects the outside air temperature may be used.

The temperature rise of the adsorbent due to the adsorption heat and earlier heating start timing of the adsorbent may be combined together. That is, when the pressure P of the reactor 1 reaches the heating start pressure P2, the adsorbent may start to be heated by the heat exchanger 7, and in addition, when the pressure P of the reactor 1 reaches the predetermined pressure P1 and the deaeration is completed, CO₂ may be supplied from the storage tank 6 to the reactor 1. In this case, the temperature rise of the adsorbent can be further accelerated. In addition, after the deaeration is completed, CO₂ is supplied. Therefore, it becomes possible to prevent the oxidation deterioration of the adsorbent with certainty.

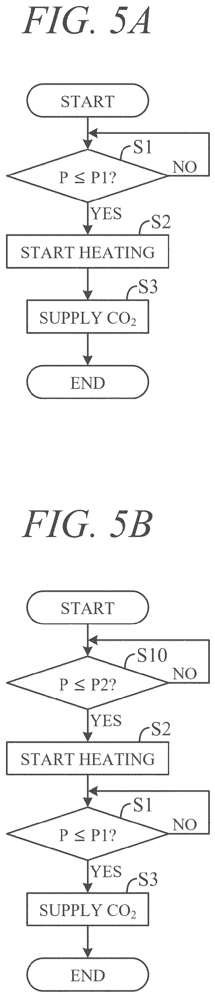

FIGS. 5A and 5B each are a flowchart illustrating an example of processing in the deaeration step performed by the controller 10 in FIG. 2.

FIG. 5A illustrates an example of processing in a case where only the temperature of the adsorbent is raised due to the adsorption heat. As illustrated in FIG. 5A, after the deaeration step starts, the controller 10 determines in S1 (S: processing step) whether the pressure P of the reactor 1 that has been detected by the pressure sensor 8 is equal to or lower than the predetermined pressure P1. The processing of S1 is repeated until an affirmative determination is made. In a case where the pressure P of the reactor 1 reaches the predetermined pressure P1 and the affirmative determination is made in S1, the controller 10 controls the heat exchanger 7 to start heating the adsorbent in S2, and controls the supply control valve 9a to supply the reactor 1 with CO₂ in S3.

FIG. 5B illustrates an example of processing in a case where the temperature of the adsorbent is raised due to the adsorption heat and earlier heating start timing of the adsorbent is applied. As illustrated in FIG. 5B, after the deaeration step starts, the controller 10 determines in S10 whether the pressure P of the reactor 1 that has been detected by the pressure sensor 8 is equal to or lower than the heating start pressure P2. The processing of S10 is repeated until an affirmative determination is made. In a case where the pressure P of the reactor 1 reaches the heating start pressure P2 and the affirmative determination is made in S10, the controller 10 controls the heat exchanger 7 to start heating the adsorbent in S2. Next, in S1, the controller 10 determines whether the pressure P of the reactor 1 that has been detected by the pressure sensor 8 is equal to or lower than the predetermined pressure P1. The processing of S1 is repeated until an affirmative determination is made. In a case where the pressure P of the reactor 1 reaches the predetermined pressure P1 and the affirmative determination is made in S1, the controller 10 controls the supply control valve 9a to supply the reactor 1 with CO₂ in S3.

The affirmative determination may be made in S1 in FIGS. 5A and 5B under the condition that a state in which the pressure P of the reactor 1 is equal to or lower than the predetermined pressure P1 continues for a predetermined time, or the affirmative determination may be made under the condition that a state in which pressure fluctuation ΔP per unit time falls within a predetermined value ΔP0 continues for a predetermined time. In this case, it becomes possible to determine completion of the deaeration with higher accuracy, and to prevent the oxidation deterioration of the adsorbent with more certainty.

According to the embodiments of the present invention, the following operation and effects are achievable.
(1) The DAC apparatus 100 includes: the reactor 1 in which an adsorbent for adsorbing or absorbing CO₂ is provided; the atmospheric control valves 1a and 1b, which open the reactor 1 to atmosphere or seal the reactor 1 from the atmosphere; the vacuum piping 4, which connects the reactor 1 with the vacuum pump 5; the vacuum control valve 4a provided on the vacuum piping 4; the heat exchanger 7, which heats the adsorbent; and the controller 10 configured to control the atmospheric control valves 1a and 1b, the vacuum control valve 4a, and the heat exchanger 7 to alternately perform an adsorption step and a desorption step, the adsorption step opening the reactor 1 to the atmosphere, prohibiting a flow of gas to the vacuum piping 4, and adsorbing CO₂ in the atmosphere to the adsorbent, the desorption step sealing the reactor 1, permitting the flow of the gas to the vacuum piping 4, and heating the adsorbent to desorb CO₂ from the adsorbent (FIGS. 1 and 2).
   The supply piping 9 to which CO₂ is supplied is further connected with the reactor 1, and the supply control valve 9a is provided on the supply piping 9 (FIG. 1). The controller 10 further controls the supply control valve 9a to supply the reactor 1 with CO₂ in the desorption step (FIGS. 5A and 5B). In this manner, by supplying the reactor 1 with CO₂ in the desorption step to raise the temperature of the adsorbent itself due to the adsorption heat and accelerate the temperature rise, it becomes possible to shorten the time until the temperature reaches the desorption temperature T0 of CO₂, that is, the time necessary for the desorption step, so that the CO₂ recovery efficiency of the entire apparatus can be improved.
(2) The DAC apparatus 100 further includes the pressure sensor 8, which detects the pressure P of the reactor 1 (FIGS. 1 and 2). When the adsorption step ends, the controller 10 performs the deaeration step of sealing the reactor 1 and also permitting the flow of gas to the vacuum piping 4 to reduce the pressure P of the reactor 1 to a predetermined pressure P1. When the pressure P that has been detected by the pressure sensor 8 reaches the predetermined pressure P1 and deaeration is completed, the supply control valve 9a is controlled to supply the reactor 1 with CO₂. In this manner, the deaeration is completed, based on the actual pressure P of the reactor 1. After the deaeration is completed, CO₂ is supplied to raise the temperature of the adsorbent due to the adsorption heat, so that the oxidation deterioration of the adsorbent can be prevented with certainty.
(3) The DAC apparatus 100 further includes the storage tank 6, which is connected with the exhaust port of the vacuum pump 5, and which stores CO₂ that has been recovered (FIG. 1). The vacuum piping 4 connects the reactor 1 with the intake port of the vacuum pump 5 (FIG. 1). The supply piping 9 connects the storage tank 6 with the reactor 1 (FIG. 1). In this manner, by supplying the reactor 1 with CO₂ stored in the storage tank 6, a simple and waste-free apparatus configuration is achievable.
(4) When the adsorption step ends, the controller 10 seals the reactor 1, and also permits the flow of the gas to the vacuum piping 4 to reduce the pressure P of the reactor 1 to the predetermined pressure P1, and controls the heat exchanger 7 to start heating the adsorbent before the pressure P of the reactor 1 reaches the predetermined pressure P1 in such a manner that the temperature of the adsorbent starts to rise at a timing when the pressure P of the reactor 1 reaches the predetermined pressure P1 (FIG. 5B). In this manner, the adsorbent starts to be heated before the deaeration is completed, and the temperature of the adsorbent is raised at the timing when the deaeration step is completed, so that the temperature rise of the adsorbent is accelerated, the time necessary for the desorption step is shortened, and the CO₂ recovery efficiency of the entire apparatus can be improved.
(5) The DAC apparatus 100 further includes the pressure sensor 8, which detects the pressure P of the reactor 1 (FIGS. 1 and 2). When the pressure P that has been detected by the pressure sensor 8 reaches the predetermined heating start pressure P2, which is higher than the predetermined pressure P1, the controller 10 controls the heat exchanger 7 to start heating the adsorbent (FIG. 5B). In this case, it becomes possible to start heating the adsorbent at an appropriate timing in the deaeration step.

The above embodiment can be combined as desired with one or more of the aforesaid modifications. The modifications can also be combined with one another.

According to the present invention, it becomes possible to accelerate the temperature rise of the adsorbent, so that the CO₂ recovery efficiency can be improved.

Above, while the present invention has been described with reference to the preferred embodiments thereof, it will be understood, by those skilled in the art, that various changes and modifications may be made thereto without departing from the scope of the appended claims.

DAC apparatus (100) configured to recover CO₂ in atmosphere, includes: adsorption-chamber (1) provided with adsorbent for adsorbing or absorbing CO₂; atmospheric-control-valve (1a,1b) configured to open adsorption-chamber to atmosphere or to seal adsorption-chamber from atmosphere; vacuum-piping (4) connecting adsorption-chamber with vacuum-pump (5); vacuum-control-valve (4a) provided on vacuum-piping; heating-device (7) configured to heat adsorbent; and controller (10) configured to control atmospheric-control-valve, vacuum-control-valve, and heating-device to alternately perform adsorption-step and desorption-step, adsorption-step opening adsorption-chamber to atmosphere, prohibiting flow of gas to vacuum-piping, and adsorbing CO₂ in atmosphere to adsorbent, desorption-step sealing adsorption-chamber, permitting flow of gas to vacuum-piping, and heating adsorbent to desorb CO₂ from adsorbent. Supply-piping (9) to which CO₂ is supplied is further connected with adsorption-chamber. Supply-control-valve (9a) is provided on Supply-piping. Controller further controls Supply-control-valve to supply adsorption-chamber with CO₂ in desorption-step.

## Claims

1. A DAC apparatus (100) configured to recover CO₂ in the atmosphere, comprising:
an adsorption chamber (1) provided with an adsorbent for adsorbing or absorbing CO₂;
an atmospheric control valve (1a,1b) configured to open the adsorption chamber (1) to the atmosphere or to seal the adsorption chamber (1) from the atmosphere;
a vacuum piping (4) connecting the adsorption chamber (1) with a vacuum pump (5);
a vacuum control valve (4a) provided on the vacuum piping (4);
a heating device (7) configured to heat the adsorbent; and
a controller (10) configured to control the atmospheric control valve (1a,1b), the vacuum control valve (4a), and the heating device (7) to alternately perform an adsorption step and a desorption step, the adsorption step opening the adsorption chamber (1) to the atmosphere, prohibiting a flow of gas to the vacuum piping (4), and adsorbing CO₂ in the atmosphere to the adsorbent, the desorption step sealing the adsorption chamber (1), permitting the flow of the gas to the vacuum piping (4), and heating the adsorbent to desorb CO₂ from the adsorbent, wherein
a supply piping (9) to which CO₂ is supplied is further connected with the adsorption chamber (1), wherein
a supply control valve (9a) is provided on the supply piping (9), wherein
the controller (10) further controls the supply control valve (9a) to supply the adsorption chamber (1) with CO₂ in the desorption step.

2. The DAC apparatus (100) according to claim 1, further comprising:
a pressure sensor (8) configured to detect a pressure of the adsorption chamber (1), wherein
the controller (10):
reduces the pressure of the adsorption chamber (1) to a predetermined pressure by sealing the adsorption chamber (1) and permitting the flow of the gas to the vacuum piping (4) when the adsorption step ends; and
controls the supply control valve (9a) to supply the adsorption chamber (1) with CO₂ when the pressure detected by the pressure sensor (8) reaches the predetermined pressure.

3. The DAC apparatus (100) according to claim 1 or 2, further comprising:
a storage tank (6) connected with an exhaust port of the vacuum pump (5) and configured to store recovered CO₂, wherein
the vacuum piping (4) connects the adsorption chamber (1) with an intake port of the vacuum pump (5), wherein
the supply piping (9) connects the storage tank (6) with the adsorption chamber (1).

4. A DAC apparatus (100) configured to recover CO₂ in the atmosphere, comprising:
an adsorption chamber (1) provided with an adsorbent for adsorbing or absorbing CO₂;
an atmospheric control valve (1a,1b) configured to open the adsorption chamber (1) to the atmosphere or to seal the adsorption chamber (1) from the atmosphere;
a vacuum piping (4) connecting the adsorption chamber (1) with a vacuum pump (5);
a vacuum control valve (4a) provided on the vacuum piping (4);
a heating device (7) configured to heat the adsorbent; and
a controller (10) configured to control the atmospheric control valve (1a,1b), the vacuum control valve (4a), and the heating device (7) to alternately perform an adsorption step and a desorption step, the adsorption step opening the adsorption chamber (1) to the atmosphere, prohibiting a flow of gas to the vacuum piping (4), and adsorbing CO₂ in the atmosphere to the adsorbent, the desorption step sealing the adsorption chamber (1), permitting the flow of the gas to the vacuum piping (4), and heating the adsorbent to desorb CO₂ from the adsorbent, wherein
the controller (10):
reduces a pressure of the adsorption chamber (1) to a predetermined pressure by sealing the adsorption chamber (1) and permitting the flow of the gas to the vacuum piping (4) when the adsorption step ends; and
controls the heating device (7) to start heating the adsorbent before the pressure of the adsorption chamber (1) reaches the predetermined pressure in such a manner that the temperature of the adsorbent starts to rise at a timing when the pressure of the adsorption chamber (1) reaches the predetermined pressure.

5. The DAC apparatus (100) according to claim 4, further comprising:
a pressure sensor (8) configured to detect the pressure of the adsorption chamber (1), wherein
the controller (10) controls the heating device (7) to start heating the adsorbent when the pressure detected by the pressure sensor (8) reaches a predetermined heating start pressure higher than the predetermined pressure.
